# EUROPÄISCHE PATENTSCHRIFT

(11) **EP 3 792 925 B1**
(45) Veröffentlichungstag und Bekanntmachung des Hinweises auf die Patenterteilung: **18.11.2026**
(21) Anmeldenummer: 19196763.7
(22) Anmeldetag: 11.09.2019
(51) Int. Cl.: G16H 10/60

(54) **VERFAHREN UND VORRICHTUNG ZUR DATENTECHNISCHEN KOMMUNIKATION IN EINEM NETZWERK**
METHOD AND APPARATUS FOR DATA TECHNICAL COMMUNICATION IN A NETWORK
PROCÉDÉ ET DISPOSITIF DE COMMUNICATION TECHNIQUE DES DONNÉES DANS UN RÉSEAU

(43) Veröffentlichungstag der Anmeldung: 17.03.2021
(73) Patentinhaber: Siemens Healthineers AG, 91301 Forchheim (DE)
(72) Erfinder: Brost, Alexander, 91058 Erlangen (DE)
(74) Vertreter: Siemens Healthineers Patent Attorneys

(56) Entgegenhaltungen:
- WO-A1-2013/123085
- WO-A1-2019/103913
- US-A1- 2015 356 257
- US-A1- 2016 147 945
- ANONYMOUS: "De-identification - Wikipedia", WIKIPEDIA, 20 July 2019 (2019-07-20), XP055670659, Retrieved from the Internet <URL:https://en.wikipedia.org/w/index.php?title=De-identification&oldid=907090769> [retrieved on 20200220]

## Beschreibung

Die Erfindung betrifft ein Verfahren und eine Vorrichtung zur datentechnischen Kommunikation in einem Netzwerk, sowie ein System zur datentechnischen Kommunikation.

Der Verwendung von künstlicher Intelligenz ("KI") kommt in der medizinischen Gemeinschaft eine immer größere Bedeutung zu. Im Rahmen der Medizin liefern KI-Algorithmen zusätzliche Informationen, die bisher entweder noch nicht verfügbar waren oder einen Zeitaufwand einer medizinisch geschulten Person erforderten. KI-Algorithmen erfordern in der Regel eine große Rechenleistung, so dass es zumeist vorteilhaft ist, sie als externe Dienste, z.B. in Form einer Cloud-Umgebung zur Verfügung zu stellen.

Dabei tritt jedoch das Problem auf, dass viele medizinische Einrichtungen keine durch einen KI-Algorithmus erzeugten Ergebnisse direkt in ihrem PACS oder RIS (oder EMR) haben möchten und fordern nach der Bearbeitung von Patientendaten durch einen KI-Algorithmus zunächst eine Kontrolle durch einen Menschen. Dabei ist RIS die Abkürzung für "Radiologieinformationssystem", PACS für "Picture Archiving and Communication System" (Dt.: Bildarchivierungs- und Kommunikationssystem) und EMR für "Electronic medical record" (Dt.: elektronische medizinische Akte). Ein häufig verwendeter Standard für das Datenformat ist der DICOM-Standard (DICOM = Digital Imaging and Communications in Medicine = Digitale Bildverarbeitung und Kommunikation in der Medizin).

Was die Daten betrifft, so liegen diese in der Netzwerkumgebung einer medizinischen Einrichtung häufig als PHI-Daten vor (PHI: patient health information), was bedeutet, dass Patientennamen mit den Daten verknüpft sind, also auf den Gesundheitszustand von Patienten direkt geschlossen werden kann. Demgegenüber stehen die NoPHI-Daten (anonymisierte Daten), die keinen direkten Rückschluss auf einen Patienten erlauben. Aus rechtlichen Gründen ist es oftmals so, dass keinen PHI-Daten an einen externen Dienst **(z.B.** einen Cloud-Dienst) gesendet werden dürfen, zumindest dann nicht, wenn dieser Cloud-Dienst mittels eines öffentlichen Netzwerks kontaktiert wird, wie **z.B.** über das Internet. Ein Netzwerk, in dem keine PHI-Daten verarbeitet werden dürfen, wird im Folgenden auch als ein "NoPHI-Netzwerk" bezeichnet im Unterschied zu einem medizintechnisches Netzwerk, was als "PHI-Netzwerk" angesehen wird, wo PHI-Daten verarbeitet werden dürfen. Das Internet ist beispielsweise ein NoPHI-Netzwerk.

Um das Problem der Übermittlung an ein PACS oder ein anderes medizinisches System zu lösen, haben Anbieter von KI-Diensten z.B. dedizierte Benutzeroberflächen implementiert, um die Ergebnisse zu bestätigen und abzulehnen. Dies erfordert jedoch, dass sich das sich ein Benutzer (medizinisches Fachpersonal) manuell beim KI-System anmeldet, um die Ergebnisse zu überprüfen. Der Benutzer weiß dabei nicht, ob überhaupt KI-Ergebnisse verfügbar sind. Da Daten anonymisiert in der Cloud gespeichert sein müssen (zumindest, wenn sie über ein NoPHI-Netzwerk erreichbar ist), kann der Arzt einen Patienten nicht direkt identifizieren.

Aus der Druckschrift WO 2013/123085 A1 ist ein System zum anonymisierten Upload von medizinischen Bilddaten bekannt. Aus der Druckschrift US 2002/0010679 A1 ist ein System zur verschlüsselten Verteilung von medizinischen Daten bekannt. Aus der Druckschrift US2016/147945 A1 ist ein System zum Durchführen von sicheren Überprüfungen von Details zu Patientenakten offenbart, die über mehrere Institutionen verteilt gespeichert sind.

Es ist eine Aufgabe der vorliegenden Erfindung, ein alternatives, komfortableres Verfahren und eine entsprechende Vorrichtung zur datentechnischen Kommunikation in einem Netzwerk, sowie ein System zur datentechnischen Kommunikation anzugeben, mit dem die oben beschriebenen Nachteile vermieden werden.

Diese Aufgabe wird durch ein Verfahren gemäß Patentanspruch 1, ein Verfahren gemäß Patentanspruch 8, ein Verfahren gemäß Patentanspruch 9, eine Vorrichtung gemäß Patentanspruch 10, ein System gemäß Patentanspruch 12, sowie durch ein medizintechnisches System gemäß Patentanspruch 14 gelöst.

Das erfindungsgemäßen Verfahren dient zur datentechnischen Kommunikation in einem Netzwerk, wobei dieses Netzwerk einen ersten Netzwerkbereich (dies ist ein medizintechnischer Netzwerkbereich bzw. ein PHI-Netzwerk) und einen zweiten Netzwerkbereich (dies ist ein NoPHI-Netzwerkbereich, z.B. das Internet) umfasst , an den bestimmungsgemäß Patientendaten anonymisiert gesendet werden. In dem ersten (medizintechnischen) Netzwerkbereich können Patientendaten als PHI-Daten vorliegen, da dort bestimmungsgemäß medizintechnische Standards gelten sollen. Das Verfahren umfassend die folgenden Schritte:
- Bereitstellen von medizintechnischen Patientendaten,
- Bereitstellen von Identifizierungsdaten zur Identifizierung des Patienten,
- Bereitstellen einer Kennung, welche mit den Identifizierungsdaten verknüpft ist.

Mit oder nach dem Senden der Statusmitteilung an den Benutzer werden entsprechende Ergebnisdaten vom Server an den Benutzer gesendet. Die vom Benutzer empfangenen Ergebnisdaten werden an ein Gerät im medizintechnischen Netzwerk gesendet. Zusammen mit der Kennung wird ein Token von dem Benutzer aus an den Server gesendet und ein Token zusammen mit der Statusmitteilung zurückgesendet. Das medizintechnische Netzwerk umfasst ein Element, das diesen Token dem Benutzer zuordnen kann und eine Statusmitteilung und die Ergebnisdaten in Abhängigkeit von den ihnen beigefügten Tokens zu einer bestimmten Station im medizintechnischen Netzwerk sendet.

Diese Schritte besagen zunächst, dass diese Daten vorliegen müssen. Auch wenn die Daten theoretisch von einer beliebigen Stelle stammen können, ist besonders bevorzugt, dass sie zunächst in einem medizintechnischen Netzwerk als PHI-Daten vorliegen. Dieses medizintechnische Netzwerk kann z.B. das Netzwerk eines Krankenhauses oder einer Arztpraxis sein. Die Daten können dort insbesondere in einem RIS, einem PACS, einem medizintechnischen Gerät (z.B. einem Messgerät, einem bildgebenden System oder einem sonstigen Untersuchungsgerät) vorliegen.

Medizintechnische Patientendaten sind Daten zum medizinischen Zustand des Patenten bzw. Untersuchungs- oder Messdaten. Beispielsweise umfassen diese Patientendaten Bilder (oder Rohdaten zu Bildern) numerische und/oder textuelle Daten zu Untersuchungen (z.B. EEG oder EKG-Daten) oder sonstige medizintechnische Daten, welche ausgewertet werden sollen.

Identifizierungsdaten zur Identifizierung des Patienten sind diejenigen Daten, mit denen ein Patient identifiziert werden kann. Diese Daten können z.B. Angaben der Gruppe Name, Adresse, Geburtsdatum und eine Nummer (z.B. eine Ausweisnummer) umfassen. Auch wenn die Daten durchaus Angaben umfassen können, mit denen ein Patient nicht so leicht zu identifizieren ist (z.B. eine laufende Nummer oder ein Aktenzeichen), steht dies hier nicht im Vordergrund, da die Identifizierungsdaten eine einfache Identifikation ermöglich sollen. Bevorzugt umfassen die Identifizierungsdaten nur Angaben, mit denen eine direkte Identifizierung eines Patienten möglich ist (z.B. wie oben gesagt: Daten der Gruppe Name, Adresse, Geburtsdatum und eine Ausweisnummer).

Anonymisierte Identifizierungsdaten werden hier als "Kennung" bezeichnet. Diese Kennung ist datentechnisch mit den Identifizierungsdaten verknüpft. Dies bedeutet insbesondere, dass die Kennung codierte Identifizierungsdaten darstellt. Die Kennung muss dabei so beschaffen sein, dass ein Rückschluss auf Identifizierungsdaten eines Patienten eindeutig möglich ist und dass für jeweils gleiche Identifizierungsdaten auch stets identische Kennungen erzeugt werden. Dies kann z.B. mittels einer Lookup-Tabelle oder einer eineindeutigen Funktion erreicht werden, sowie durch einen entsprechenden Algorithmus. Die Kennung dient zur Kennzeichnung der Daten in einem NoPHI-Bereich, so dass in dem Falle, in dem außerhalb des medizintechnischen Netzwerks Daten in die Hände unbefugter gelangen, diese nicht erkennen können, welchem Patienten diese Daten zuzuordnen sind. Bevorzugt wird nach Erstellen einer Kennung diese zusammen mit den entsprechenden Identifizierungsdaten in einer Datenbank (z.B. als Tabelleneintrag) abgespeichert.

Es sollte beachtet werden, dass wenn aus Identifizierungsdaten, aus denen bereits eine erste Kennung erzeugt worden ist, erneut eine (zweite) Kennung erzeugt wird, diese beiden Kennungen (in wesentlichen Teilen) identisch sein müssen oder zumindest eine Identifizierung eines Datensatzes beim Cloud-Dienst ermöglichen müssen, damit ein Patient stets korrekt erkannt werden kann. Dies kann z.B. dadurch erreicht werden, dass stets die selben Identifizierungsdaten eines Patienten auf dieselbe Art codiert bzw. anonymisiert werden, um stets dieselbe Kennung zu erzeugen oder eine Kennung mit stets identischen Teilen (z.B. einen Identifizierungsteil). Es kann aber auch vor Erzeugen der Kennung in einer vorgegebenen Tabelle stets nachgeschaut werden, ob für Identifizierungsdaten bereits eine Kennung erzeugt worden ist, im positiven Fall diese Kennung verwendet werden und im negativen Fall eine neue Kennung erzeugt werden und die Informationen dazu (z.B. Identifizierungsdaten und Kennung) in die Tabelle geschrieben werden.

Es wird an dieser Stelle angemerkt, dass die Kennung, die auf die Bereitstellung oder Eingabe von Identifizierungsdaten erzeugt wird, nicht unbedingt direkt vom Benutzer oder dem System stammen muss, die diese Identifizierungsdaten bereitstellt (aber durchaus stammen kann). Es kommt lediglich darauf an, dass der Cloud-Dienst eine Kennung erhält, die er einem Datensatz zum entsprechenden Patienten zuordnen kann. Die Kennung kann also auch von einem zwischenliegenden System (mit einer Anonymisierungseinheit, z.B. einer Lookup-Tabelle) stammen. Die Kennungen (erste Kennung zur Markierung der Daten und die zweite Kennung nach der Benutzereingabe oder Bereitstellung) sollten identische Teile aufweisen, damit eine eindeutige Zuordnung zu einem Datensatz bei dem Cloud-Dienst vorgenommen werden kann, sie können aber auch noch zusätzliche, nicht notwendigerweise identische, Informationen enthalten (wie z.B. eine laufende Nummer, eine Zufallszahl, oder ein hash-Wert).

Beispielsweise können die Patientendaten CT-Bilder eines Patienten sein, die Identifizierungsdaten, Name und Geburtsdatum des Patienten und die Kennung ein alphanumerischer Code, der über eine Lookup-Tabelle mit dem Patienten verknüpft ist.
- Senden von medizintechnischen Patientendaten und der dazugehörigen Kennung aus dem ersten (medizintechnischen) Netzwerkbereich an einen Server (z.B. einen Cloud-Dienst) im zweiten (NoPHI-) Netzwerkbereich.

Von den vorgenannten bereitgestellten Daten wird nun eine Anzahl von Datensätzen, nämlich diejenigen, die durch den Server (z.B. einen Cloud-Dienst) bearbeitet werden sollen, an den Server gesendet. Dort werden die Daten durch einen vom Server bereitgestellten Dienst (z.B. besagtem Dienst in der Cloud) bearbeitet. Bei einem Cloud-Dienst wäre der Server von der Cloud-Architektur umfasst. Im Folgenden wird der besseren Übersicht halber auch von "Dienst" gesprochen, wobei mit diesem Begriff stets auch der Server als Hardware umfasst ist. Genauso ist mit "Server" auch der Dienst umfasst, den der Server zur Verfügung stellt. Der Server ist im zweiten (No-PHI-) Netzwerkbereich angeordnet.

Ein Dienst auf dem Server (z.B. ein Cloud-Dienst) kann z.B. der Dienst "AI-Rad Companion" sein. Dieser Dienst dient der Bearbeitung der Patientendaten, insbesondere mittels einer künstlichen Intelligenz, kann aber auch ein Dienst basierend auf herkömmlichen Algorithmen sein oder ein Dienst, bei dem im Hintergrund eine Auswertung durch Menschen stattfindet. Selbstverständlich muss die Kennung mitgesendet werden, damit die bearbeiteten Patientendaten später dem richtigen Patienten zugeordnet werden können. Die Sendung der Patientendaten zusammen mit den Identifizierungsdaten kommt hier nicht infrage, da davon ausgegangen wird, dass der Dienst über ein NoPHI-Netzwerk (den zweiten Netzwerkbereich) erreichbar ist, also in einem Netzwerkbereich liegt, bei dem (z.B. nach geltenden Sicherheitsbestimmungen) nicht von einer Vertraulichkeit oder Datensicherheit ausgegangen werden kann.

Ein Cloud-Dienst (im Folgenden auch kurz als "Cloud" bezeichnet) ist eine IT-Infrastruktur, bei der z.B. Speicherplatz oder Rechenleistung und/oder eine Anwendungssoftware über ein Netzwerk zur Verfügung gestellt wird. Die Kommunikation zwischen dem Anwender und der Cloud erfolgt dabei mittels Datenschnittstellen und/oder Datenübertragungs-protokollen. Im hier vorliegenden Fall ist besonders bevorzugt, dass der Cloud-Dienst sowohl Rechenleistung als auch Anwendungssoftware zur Verfügung stellt.

Im Rahmen eines bevorzugten Verfahrens erfolgt eine Bereitstellung von Patientendaten über das Netzwerk an den Cloud-Dienst. Dieser umfasst ein Rechensystem, z.B. einen Computercluster, das in der Regel nicht den lokalen Rechner des Benutzers umfasst. Diese Cloud kann insbesondere durch die medizinische Einrichtung, die auch die medizintechnischen Systeme bereitstellt, zur Verfügung gestellt werden. Beispielsweise werden die Daten einer Bildaufnahme über ein RIS oder PACS an ein (Remote-) Rechnersystem (die Cloud) gesendet. Bevorzugt stellen das Rechensystem der Cloud, das Netzwerk sowie das medizintechnische System einen Verbund im datentechnischen Sinne dar. Das Verfahren kann dabei mittels einer Befehlskonstellation in dem Netzwerk realisiert werden. Die in der Cloud berechneten Daten ("Ergebnisdaten") werden später wieder über das Netzwerk zu dem lokalen Rechner des Anwenders gesendet (siehe unten).

Auf Seiten des Servers folgt dann eine
- Bearbeiten der Patientendaten durch den Server (z.B. den Cloud-Dienst).

Der Server bzw. der Dienst bearbeitet die Patientendaten nun nach vorgegebenen Bedingungen. Dies kann z.B. eine Auswertung von Bildern und die Markierung von auffälligen Bereichen in diesen Bildern sein. Es werden bearbeitete Patientendaten erzeugt, die im Folgenden auch als "Ergebnisdaten" bezeichnet werden. Diese Bearbeitung ist der Grund, weshalb die Daten überhaupt an den Server (z.B. an einen Cloud-Dienst) gesendet werden.
- Bereitstellung von Identifizierungsdaten oder Eingabe von Identifizierungsdaten zur Identifizierung eines Patienten durch einen Benutzer.

Hier wird nun durch einen Benutzer direkt ein Patient genannt. Beispielsweise wird der Name (oder andere Daten) des Patienten in eine Suchmaske eingetragen. Auch wenn es theoretisch möglich sein könnte, sollte in diesem Schritt keine Daten ähnlich einer Kennung eingegeben werden, sondern Identifizierungsdaten, die einen direkten Schluss auf den Patienten zulassen. Dieser Schritt kann auch automatisiert werden, z.B. durch bestimmte Richtlinien und Benutzerverwaltungen (z.B. könnte der Dienst "Active Directory" dazu verwendet werden). Active Directory ist eine Benutzerauthentifizierung (Login) für einen Mediziner. Damit wird gewährleistet, dass nur autorisierte Benutzer Zugriff auf den Cloud-Dienst bekommen. Ein Mediziner kann nach einen Patienten direkt suchen, es müsste aber in der Praxis vom Benutzersystem die passende Kennung erzeugt werden, wenn nicht eine zentrale Stelle bemüht wird (s.o.). Kennt das Benutzersystem (oder der Benutzer selber) die Kennung nicht, kann die Generierung der Kennung mittels Active Directory automatisch erfolgen, wie z.B. oben genauer beschrieben wird, indem beispielsweise eine zentrale Anonymisierungseinheit im medizintechnischen Netzwerk verwendet wird.
- Ermitteln einer mit diesen Identifizierungsdaten verknüpften Kennung.

Die Identifizierungsdaten sollten nicht an den zweiten Netzwerkbereich gesendet werden. Der Dienst könnte auch mit diesen Identifizierungsdaten nichts anfangen, da er nur Kennungen vorliegen hat. Daher müssen die Identifizierungsdaten zunächst als Kennung codiert werden. Dies geschieht nach demselben Prinzip wie die vorgenannten Kennungen ermittelt worden sind (s.o.). Dabei hat jeder Patient stets dieselbe Kennung. Dies kann z.B. dadurch erreicht werden, dass in dem Fall, dass die hier genannten Identifizierungsdaten mit den vorgenannten Identifizierungsdaten verglichen werden (z.B. mittels einer Lookup-Tabelle) und bei Übereinstimmung die diesbezügliche oben genannte Kennung hier verwendet wird. Liegt keine Übereinstimmung vor, kann die Kennung eine leere Menge oder ein Fehlercode sein. Selbstverständlich ist es auch möglich, die Kennung aus den Identifizierungsdaten mittels einer vorgegebenen Funktion oder mittels eines vorgegebenen Algorithmus zu ermitteln, wobei die gleiche Funktion bzw. der gleiche Algorithmus verwendet wird, wie bei der oben genannten Kennung, so dass für jeden Patienten stets dieselbe Kennung erzeugt wird.
- Automatisches Senden der Kennung an den Server (z.B. den Cloud-Dienst).

Die Kennung wird nun an den Server bzw. den Dienst über das Netzwerk gesendet.

Auf Seiten des Servers erfolgt nun ein
- Ermitteln des Status der Bearbeitung von mit dieser Kennung verbundenen Patientendaten, und Erstellung einer entsprechenden Statusmitteilung durch den Server (z.B. den Cloud-Dienst).

Da der Server bzw. der Dienst die Patientendaten zu Bearbeitung zusammen mit der Kennung erhalten hat, ist es ein leichtes, zu ermitteln, wie der Bearbeitungsstatus der betreffenden Patientendaten ist. Im Grunde gibt es eine übersichtliche Anzahl von Möglichkeiten, nämlich z.B.: "Nicht vorhanden", "Noch nicht bearbeitet", "Bearbeitung läuft" (ggf. mit einer Angabe des Fortschritts oder der bearbeiteten Menge), "Bearbeitung abgeschlossen" und "Fehler bei der Bearbeitung". Die Statusmitteilung kann einen alphanumerischen Code enthalten, der einer Mitteilung standardmäßig zugeordnet werden kann, die Statusmitteilung kann aber auch einen Text enthalten, z.B. eine der oben genannten Statusmeldungen.

Nach der Ermittlung des Status erfolgt auf Seiten des Servers ein
- Senden der Statusmitteilung an den Benutzer.

Die Statusmitteilung wird dabei über das Netzwerk an den Benutzer gesendet.
- Empfangen einer Statusmitteilung (S) des Servers (AI) und Weitergabe der Statusmitteilung (S) an den Benutzer.

Die vom Server gesendete Statusmeldung wird auf Benutzerseite empfangen und dem Benutzer bevorzugt angezeigt (ggf. nach Zuordnung eines alphanumerischen Codes der Statusmitteilung zu einer standardmäßigen Mitteilung). Bevorzugt wird dabei die Kennung wieder den Identifizierungsdaten des Patienten zugeordnet, so dass der Benutzer sofort weiß, für welchen Patienten diese Statusmitteilung ist (z.B. wenn mehrere Patienten gleichzeitig abgefragt worden sind).

Aus sicherheitstechnischen Gründen ist bevorzugt, dass vor diesem Schritt (aber bevorzugt bereits vor der oben genannten Eingabe) eine Benutzerauthentifizierung erfolgt, so dass keine unautorisierten Personen Patientendaten einsehen können. Die Benutzerauthentifizierung kann bei Zugriff auf das Netzwerk erfolgen, bei der Eingabe von Identifizierungsdaten oder im Rahmen des hier genannten Schrittes. Es ist von Vorteil, wenn auch in dem Fall, dass die klinischen Daten anonymisiert auf dem Server **(z.B.** in der Cloud) vorliegen, dennoch Sorge getragen wird, dass Unbefugte keinen Zugriff bekommen.

Aus dem gleichen Grund sollte die gesamte Kommunikation verschlüsselt ablaufen, es sei denn, das gesamte Netzwerk ist sicher vor dem unerlaubten Zugriff Dritter geschützt. Zumindest sollte dort, wo Dritte Zugriff auf die Daten haben können, die Kommunikation verschlüsselt erfolgen.

Ein erfindungsgemäßes (benutzerseitiges) Verfahren zur datentechnischen Kommunikation in einem Netzwerk mit einem ersten Netzwerkbereich und einem zweiten Netzwerkbereich, an den bestimmungsgemäß Patientendaten anonymisiert gesendet werden, kann mit dem im Folgenden beschriebenen serverseitigen Verfahren Hand in Hand arbeiten. Damit überhaupt Daten bearbeitet werden und eine entsprechende Mitteilung überhaupt erstellt werden kann, sollten die folgenden Schritte im Vorfeld erfolgen:
- Bereitstellen von medizintechnischen Patientendaten,
- Bereitstellen von Identifizierungsdaten zur Identifizierung eines Patienten,
- Bereitstellen einer Kennung, welche mit den Identifizierungsdaten verknüpft ist,
- Senden von medizintechnischen Patientendaten und der dazugehörigen Kennung aus dem ersten Netzwerkbereich an einen Server im zweiten Netzwerkbereich (und selbstverständlich eine Bearbeitung der Patientendaten durch den Server). Diese Schritte können auch im Rahmen des (benutzerseitigen) Verfahrens ablaufen, es ist jedoch nicht unbedingt notwendig, z.B. wenn der Benutzer über einen Terminal von außen (von außerhalb des ersten (medizintechnischen) Netzwerks auf den Server zugreift. Es sei der Klarheit halber nochmals erwähnt, dass der Benutzer sich nicht unbedingt im ersten (medizintechnischen) Netzwerk befinden muss, dies jedoch bevorzugt ist.

Das (benutzerseitige) Verfahren umfasst die folgenden Schritte:
- Bereitstellung von Identifizierungsdaten oder Eingabe von Identifizierungsdaten zur Identifizierung eines Patienten durch einen Benutzer,
- Ermitteln einer mit diesen Identifizierungsdaten verknüpften Kennung,
- automatisches Senden der Kennung an den Server,
- Empfangen einer Statusmitteilung des Servers und Weitergabe der Statusmitteilung an den Benutzer.

Mit oder nach dem Senden der Statusmitteilung an den Benutzer werden entsprechende Ergebnisdaten vom Server an den Benutzer gesendet. Die vom Benutzer empfangenen Ergebnisdaten werden an ein Gerät im medizintechnischen Netzwerk gesendet. Zusammen mit der Kennung wird ein Token von dem Benutzer aus an den Server gesendet und ein Token zusammen mit der Statusmitteilung zurückgesendet. Das medizintechnische Netzwerk umfasst ein Element, das diesen Token dem Benutzer zuordnen kann und eine Statusmitteilung und die Ergebnisdaten in Abhängigkeit von den ihnen beigefügten Tokens zu einer bestimmten Station im medizintechnischen Netzwerk sendet.

Ein erfindungsgemäßes (serverseitiges) Verfahren zur datentechnischen Kommunikation in einem Netzwerk mit einem ersten Netzwerkbereich und einem zweiten Netzwerkbereich, an den bestimmungsgemäß Patientendaten anonymisiert gesendet werden, welches mit den oben aufgeführten benutzerseitigen Verfahren Hand in Hand arbeiten kann, umfasst die folgenden Schritte:
- Empfangen von medizintechnischen Patientendaten und einer dazugehörigen Kennung aus dem ersten Netzwerkbereich durch einen Server im zweiten Netzwerkbereich,
- Bearbeiten der Patientendaten (die oben an den Server gesandt worden sind) durch den Server,
- Empfangen einer von einem Benutzer gesendeten Kennung durch den Server,
- Ermitteln des Status der Bearbeitung von mit dieser Kennung verbundenen Patientendaten, und Erstellung einer entsprechenden Statusmitteilung durch den Server,
- Senden der Statusmitteilung an den Benutzer.

Mit oder nach dem Senden der Statusmitteilung an den Benutzer werden entsprechende Ergebnisdaten vom Server an den Benutzer gesendet. Die vom Benutzer empfangenen Ergebnisdaten werden an ein Gerät im medizintechnischen Netzwerk gesendet. Zusammen mit der Kennung wird ein Token von dem Benutzer aus an den Server gesendet und ein Token zusammen mit der Statusmitteilung zurückgesendet. Das medizintechnische Netzwerk umfasst ein Element, das diesen Token dem Benutzer zuordnen kann und eine Statusmitteilung und die Ergebnisdaten in Abhängigkeit von den ihnen beigefügten Tokens zu einer bestimmten Station im medizintechnischen Netzwerk sendet.

Die erfindungsgemäße Vorrichtung dient zur datentechnischen Kommunikation in einem Netzwerk mit einem ersten (medizintechnischen) Netzwerkbereich und einem zweiten (NoPHI-) Netzwerkbereich, an den bestimmungsgemäß Patientendaten anonymisiert gesendet werden. Dabei basiert die Kommunikation auf medizintechnischen Patientendaten, Identifizierungsdaten zur Identifizierung des Patienten und einer mit den Identifizierungsdaten verknüpften Kennung. Die Vorrichtung umfasst die folgenden Komponenten:
- Eine Eingabeschnittstelle ausgelegt zur Eingabe von Identifizierungsdaten zur Identifizierung eines Patienten durch einen Benutzer. Dies kann z.B. eine Tastatur, aber auch ein Terminal sein.
- Eine Identifizierungseinheit ausgelegt zum Ermitteln einer mit diesen Identifizierungsdaten verknüpften Kennung. Diese Identifizierungseinheit sollte Zugriff auf diejenige Komponente haben, welche aus Identifizierungsdaten eine Kennung erzeugt oder ein Modul zur Erzeugung einer Kennung aus Identifizierungsdaten wie oben im Rahmen des Verfahrens beschrieben.
- Eine Datenschnittstelle ausgelegt zum Senden der Kennung an den Server (z.B. den Cloud-Dienst). Diese kann eine normale, wohlbekannte Datenschnittstelle sein, insbesondere wie sie zur Kommunikation aus einem medizintechnischen Netzwerk heraus verwendet wird.
- Eine Datenschnittstelle ausgelegt zum Empfang einer Statusmitteilung des Servers (z.B. des Cloud-Dienstes) und zur Weitergabe der Statusmitteilung an den Benutzer. Diese kann eine normale, wohlbekannte Datenschnittstelle sein, insbesondere wie sie zur Kommunikation aus einem medizintechnischen Netzwerk heraus verwendet wird. Insbesondere ist sie dieselbe Datenschnittstelle wie vorangehend beschrieben, welche für eine bidirektionale Kommunikation ausgelegt ist.

Die Vorrichtung ist dabei dazu ausgelegt, mit einem Gerät oder Dienst im medizintechnischen Netzwerk zu kommunizieren. Mit oder nach dem Senden der Statusmitteilung an den Benutzer werden entsprechende Ergebnisdaten vom Server an den Benutzer gesendet. Die vom Benutzer empfangenen Ergebnisdaten werden an ein Gerät im medizintechnischen Netzwerk gesendet. Zusammen mit der Kennung wird ein Token von dem Benutzer aus an den Server gesendet und ein Token zusammen mit der Statusmitteilung zurückgesendet. Das medizintechnische Netzwerk umfasst ein Element, das diesen Token dem Benutzer zuordnen kann und eine Statusmitteilung und die Ergebnisdaten in Abhängigkeit von den ihnen beigefügten Tokens zu einer bestimmten Station im medizintechnischen Netzwerk sendet.

Die Vorrichtung ist bevorzugt zur Ausführung von Schritten des erfindungsgemäßen (benutzerseitigen) Verfahrens ausgestaltet, welche innerhalb des medizintechnischen Netzwerks und/oder am System des Benutzers erfolgen.

Ein entsprechendes erfindungsgemäßes Serversystem zur datentechnischen Kommunikation in einem Netzwerk mit einem ersten Netzwerkbereich und einem zweiten Netzwerkbereich, an den bestimmungsgemäß Patientendaten anonymisiert gesendet werden, welches mit den oben aufgeführten (benutzerseitigen) Verfahren Hand in Hand arbeiten kann, umfasst die folgenden Komponenten:
- Empfangen von medizintechnischen Patientendaten und einer dazugehörigen Kennung aus dem ersten Netzwerkbereich an einen Server im zweiten Netzwerkbereich,
- Bearbeiten der Patientendaten (die oben an den Server gesandt worden sind) durch den Server,
- Empfangen einer von einem Benutzer gesendeten Kennung durch den Server,
- Ermitteln des Status der Bearbeitung von mit dieser Kennung verbundenen Patientendaten, und Erstellung einer entsprechenden Statusmitteilung durch den Server,
- Senden der Statusmitteilung an den Benutzer.

Eine erfindungsgemäßes System zur datentechnischen Kommunikation umfasst die folgenden Komponenten:
- Ein Netzwerk umfassend einen ersten (medizintechnischen) Netzwerkbereich und einen zweiten (NoPHI-) Netzwerkbereich, an den bestimmungsgemäß Patientendaten anonymisiert gesendet werden.
- Eine Einheit ausgelegt zum Bereitstellen von medizintechnischen Patientendaten, von Identifizierungsdaten zur Identifizierung des Patienten. Diese Einheit kann z.B. ein PACS oder ein medizintechnisches bildgebendes System und/oder ein RIS sein.
- Eine Einheit ausgelegt zum Bereitstellen einer Kennung, welche mit den Identifizierungsdaten verknüpft ist. Dies kann z.B. eine Anonymisierungseinheit zur automatischen Erstellung der Kennung aus Identifizierungsdaten eines Patienten sein. Die Anonymisierung kann insbesondere nach einer vorgegebenen Tabelle, einem vorgegebenen Algorithmus oder einer vorgegebenen Funktion erfolgen.
- Eine Datenschnittstelle ausgelegt zum Senden von medizintechnischen Patientendaten und der dazugehörigen Kennung aus dem ersten (medizintechnischen) Netzwerkbereich an einen Server (z.B. einen Cloud-Dienst). Diese Netzwerkschnittstelle sollte besondere sicherheitstechnische Anforderungen erfüllen, wie sie typischerweise bei einer Kommunikation eines medizintechnischen Netzwerks mit einem als unsicher anzusehenden Netzwerk erfolgen muss.
- eine erfindungsgemäße Vorrichtung und bevorzugt auch ein erfindungsgemäßes Serversystem.

Ein erfindungsgemäßes medizintechnisches System (bzw. Gerät), insbesondere in Form eines Systems (Gerätes) der Gruppe PACS, RIS, KIS ("Krankenhausinformationssystem"), EMR, medizintechnisches Messsystem, medizintechnisches Bildgebungssystem und Therapieplanungssystem, umfasst eine erfindungsgemäße Vorrichtung (und bevorzugt auch ein erfindungsgemäßes Serversystem) oder ist zur Einbindung in ein erfindungsgemäßes System ausgelegt.

Bevorzugt ist das medizintechnische System zur Ausführung der Verfahrensschritte des erfindungsgemäßen Verfahrens ausgestaltet, die nicht im oder durch den Server (z.B. den Cloud-Dienst) ablaufen. Dies sind:
- Bereitstellen von medizintechnischen Patientendaten,
- Bereitstellen von Identifizierungsdaten zur Identifizierung des Patienten,
- Bereitstellen einer Kennung, welche mit den Identifizierungsdaten verknüpft ist,
- Senden von medizintechnischen Patientendaten und der dazugehörigen Kennung aus dem ersten (medizintechnischen) Netzwerkbereich an einen Server (z.B. einen Cloud-Dienst) im zweiten (NoPHI-) Netzwerkbereich,
- Bereitstellung von Identifizierungsdaten oder Eingabe von Identifizierungsdaten zur Identifizierung eines Patienten durch einen Benutzer,
- Ermitteln einer mit diesen Identifizierungsdaten verknüpften Kennung,
- automatisches Senden der Kennung an den Server (z.B. den Cloud-Dienst).

Ein Großteil der zuvor genannten Komponenten der Vorrichtung bzw. des Serversystems bzw. des Systems (insbesondere die vorgenannten Verfahrensschritte, die nicht im oder durch den Server, z.B. den Cloud-Dienst, ablaufen), können ganz oder teilweise in Form von Softwaremodulen in einem Prozessor einer entsprechenden Vorrichtung bzw. eines Serversystems bzw. in einem System realisiert werden. Eine weitgehend softwaremäßige Realisierung hat den Vorteil, dass auch schon bisher verwendete Vorrichtungen bzw. Systeme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem bzw. eine Netzwerkkomponente ladbar ist, mit Programmabschnitten, um alle Schritte des erfindungsgemäßen Verfahrens auszuführen, wenn das Programm in dem Rechensystem bzw. der Netzwerkkomponente ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zum Rechensystem bzw. zur Netzwerkkomponente und/oder zur Speicherung an oder in dem Rechensystem bzw. der Netzwerkkomponente kann ein computerlesbares Medium, z.B. ein Memorystick, eine Festplatte oder ein sonstiger transportabler oder fest eingebauter Datenträger dienen, auf welchem die von einem Rechensystem bzw. einer Netzwerkkomponente einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Mit oder nach dem Senden der Statusmitteilung an den Benutzer werden die bearbeiteten Patientendaten (Ergebnisdaten) vom Server (z.B. dem Cloud-Dienst) an den Benutzer gesendet. Der Benutzer kann die Ergebnisdaten automatisch (sogar zusammen mit der Statusmitteilung) erhalten, es ist jedoch bevorzugt, dass der Benutzer die Ergebnisdaten nach einer Interaktion seinerseits erhält, z.B. nach einer Anfrage, einer Bestätigung oder einem Klick auf einen Link.

Auf der Benutzerseite werden also bevorzugt mit oder nach dem Empfang der Statusmitteilung entsprechende Ergebnisdaten empfangen (die vom Server an den Benutzer gesendet wurden), insbesondere nach einer Interaktion durch den Benutzer.

Der Benutzer kann die Ergebnisdaten dann überprüfen und die überprüften Ergebnisdaten danach in einem Gerät in dem medizintechnischen Netzwerk abspeichern. Die vom Nutzer empfangenen bearbeiteten Patientendaten werden also an ein Gerät im medizintechnischen Netzwerk gesendet, insbesondere ein PACS oder RIS. Die Abspeicherung erfolgt dabei bevorzugt nach einer Interaktion durch den Benutzer (z.B. einer Bestätigung).

Gemäß einem bevorzugten Verfahren werden die medizintechnischen Patientendaten und die Kennung aus dem medizintechnischen Netzwerk an den Server (z.B. den Cloud-Dienst) gesendet, bevorzugt von einem medizintechnischen (Bildgebungs)-System, einem PACS, einem RIS, einem KIS, einer EMR und/oder einem Therapieplanungssystem. Bei dieser Ausführungsform wird sichergestellt, dass die Patientendaten aus derjenigen medizinischen Einrichtung stammen, zu der das medizintechnische Netzwerk gehört, oder von an dem medizintechnischen Netzwerk angeschlossenen Stellen.

Gemäß einem bevorzugten Verfahren greift der Benutzer über ein Gerät und/oder eine Software-Anwendung, z.B. einen PACS-Viewer, auf das medizintechnische Netzwerk zu. Die Kommunikation zwischen Server (z.B. dem Cloud-Dienst) und Benutzer erfolgt dabei über das medizintechnische Netzwerk. Dadurch wird sichergestellt, dass die Kommunikation ausschließlich über das medizintechnische Netzwerk erfolgt und nicht von einer beliebigen Stelle aus über Internet direkt auf den Server (z.B. den Cloud-Dienst) zugegriffen werden kann.

Gemäß einem bevorzugten Verfahren ist die Kommunikation zwischen dem ersten (medizintechnischen) Netzwerkbereich und dem Server (z.B. dem Cloud-Dienst) verschlüsselt und erfolgt bevorzugt mittels einer Software zum Fernzugriff auf Software-Anwendungen, z.B. der Software "VPN" oder "teamplay". Dabei erfolgt bevorzugt eine, insbesondere verschlüsselte, Kommunikation zwischen dem Benutzer und dem Server (z.B. dem Cloud-Dienst) besonders bevorzugt über eine Software zum Fernzugriff auf Software-Anwendungen. Ein Benutzer loggt sich also zunächst in das medizintechnische Netzwerk ein. Eine solche Verschlüsselung sollte bevorzugt mit einer Nutzerauthentifizierung einhergehen. Auch wenn durch die Kennung der Patient anonymisiert worden ist, hat eine solche Verschlüsselung wichtige Vorteile. So kann eine effektive Verschlüsselung z.B. unzulässige Änderungen der Patientendaten von Angreifern des Datenstroms ("Man in the Middle") effektiv verhindern bzw. aufdecken.

Gemäß einem bevorzugten Verfahren wird die Ermittlung einer Kennung anhand von Identifizierungsdaten mittels einer Lookup-Tabelle, einem Algorithmus oder einer eineindeutigen Funktion durchgeführt. Nochmals wird hier klargestellt, dass für jeden Patienten nur eine einzige Kennung vorliegen sollte. Diese sollte stets erzeugt werden, wenn die Identifizierungsdaten dieses Patienten eingegeben oder für eine Sendung an den Server (z.B. den Cloud-Dienst) bereitgestellt werden. Somit sollte die Kennung sowohl für das Senden der Patientendaten an den Dienst als auch bei späterer Eingabe bzw. Bereitstellung der Identifizierungsdaten dasselbe Verfahren zur Erstellung der Kennung aus Identifizierungsdaten verwendet werden.

Gemäß einem bevorzugten Verfahren umfasst die Statusmitteilung eine Information über den Zustand der Bearbeitung durch den Server (z.B. den Cloud-Dienst). Diese kann wie vorangehend gesagt ein alphanumerischer Code sein oder eine textuelle Mitteilung, z.B. "Daten nicht vorhanden", "in Bearbeitung", "Fertig", "Fehler". Im Falle einer fertigen Bearbeitung der Patientendaten umfasst die Statusmitteilung bevorzugt zusätzlich Informationen über ein Ergebnis. Beispielsweise kann bei der Auswertung eines CT-Bildes angegeben werden, welche Anomalien bzw. Pathologien gefunden worden sind. Es ist bevorzugt dass diese Ergebnisse in Form einer Zusammenfassung der wichtigsten Punkte erfolgen. Die Statusmitteilung wird besonders bevorzugt an den Benutzer ausgegeben, insbesondere in Form einer standardisierten Wiedergabe.

Es ist bevorzugt, dass die Statusmitteilung zusätzlich Daten umfasst, welche die Art oder das Format der Wiedergabe angeben, z.B. die Größe des Ausgabefensters, die Hintergrund- und/oder Schriftfarbe, die Schriftgröße oder Daten zu akustischen Effekten. Auch wenn die Ausgabe an sich auf dem Benutzersystem programmiert sein kann, z.B. in Form einer Angabe in einem Programm oder einer App, ist es bevorzugt, dass auf Ausgabeobjekte in dem Betriebssystem oder Bibliotheken des Benutzersystems oder auf Darstellungsfunktionen in einem PACS oder RIS zugegriffen wird, welche mit den zusätzlichen Daten formatiert werden.

Eine bevorzugte Vorrichtung umfasst eine Anonymisierungseinheit zur automatischen Erstellung der Kennung aus Identifizierungsdaten eines Patienten, insbesondere nach einer vorgegebenen Tabelle, einem vorgegebenen Algorithmus oder einer vorgegebenen Funktion. Auch wenn es denkbar ist, dass bei einer ausreichenden Verschlüsselung der Daten, eine Anonymisierung nicht stattfindet, bringt dies jedoch Einbußen der Sicherheit mit sich. Um zu gewährleisten, dass eine manuelle Re-Identifikation des Patienten mit Sicherheit von unbefugten Dritten nicht durchgeführt werden kann, muss die Anonymisierung und Re-Identifikation so erfolgen, dass dies von außen nicht eingesehen werden kann. Daher wird die Kennung durch eine Anonymisierungseinheit erzeugt, die besonders bevorzugt im medizintechnischen Netzwerk positioniert ist.

Bevorzugt kann das Senden der zu bearbeitenden Daten an den Server (z.B. den Cloud-Dienst) von der Vorrichtung initiiert oder gesteuert werden. Es ist alternativ oder zusätzlich möglich, im Netzwerk oder der Vorrichtung eine Applikation (ein Programm) anzuwenden, welches den Netzwerkverkehr steuert (z.B. "VPN", "teamplay", insbesondere den "teamplay_Receiver") und als Transfermechanismus zum Dienst wirkt. Dieses Programm kann durchaus auf einer anderen physikalischen Recheneinheit laufen als andere Komponenten der Vorrichtung. Es wäre aber denkbar, dass vor allem bei kleinen Kliniken oder Arztpraxen die Softwarekomponenten der Vorrichtung auf einem einzigen Rechner laufen.

Bei einer bevorzugten Vorrichtung ist das Gerät oder der Dienst im medizintechnischen Netzwerk ein Dienst oder Gerät der Gruppe PACS, RIS, KIS, EMR, medizintechnisches Messsystem, medizintechnisches Bildgebungssystem und Therapieplanungssystem.

Ein bevorzugtes System ist dazu ausgelegt, dass die Vorrichtung über das medizintechnische Netzwerk mit dem Server (z.B. dem Cloud-Dienst) kommuniziert. Dazu ist es bevorzugt , dass sich die Vorrichtung (bzw. der an der Vorrichtung arbeitende Benutzer) sich zunächst in das medizintechnische Netzwerk einloggt.

Es ist bevorzugt, dass das System bzw. das Verfahren dazu ausgelegt ist, eine geographisch definierte Weitergabe der über das Netzwerk gesandten Daten vorzugeben und/oder Daten nur an eine klar definierte Menge von Servern zu senden. Dies dient der Einhaltung möglicher gesetzlicher Regelungen, die verbieten, dass medizinisch relevante Daten die Staatsgrenzen verlassen. Beispielsweise umfasst das System eine Positivliste von Netzwerkschnittstellen (z.B. Routern und/oder Servern), an die Daten gesendet werden dürfen und ist besonders bevorzugt so ausgelegt, dass keine Daten an andere Netzwerkdienste gesendet werden können. Für ein bevorzugtes Verfahren gilt entsprechendes. Neben der Verwendung der vorangehend beschriebenen Positivliste sind auch die Möglichkeiten des Geoblockings und/oder der Response-Time Messung bevorzugt.

Ein Problem in der Praxis kann das Auffinden des Rechensystems sein, an das die Statusmitteilung gesendet werden soll. In einem klinischen Umfeld existieren mitunter viele kleinere Netzwerke, die untereinander durch Firewalls oder andere Sicherheitsmechanismen getrennt sind. Der Dienst selber hat zwar eine Kennung, weiß jedoch nicht, von welchem Rechensystem diese Kennung geschickt worden ist. Daher wird zusammen mit der Kennung ein Token von dem Benutzer aus (der Recheneinheit, an der sich der Benutzer eingeloggt hat) an den Dienst gesendet und zusammen mit der Statusmitteilung zurückgesendet. Ein solcher Token wird hier auch zur besseren Kennzeichnung als "Dienst-Token" bezeichnet. Das medizintechnische Netzwerk umfasst dabei ein Element, z.B. einen Proxy-server oder einen Router, das diesen Token dem Benutzer zuordnen kann und eine Statusmitteilung in Abhängigkeit von den ihnen beigefügten Tokens zu einer bestimmten Station im medizintechnischen Netzwerk (dem Rechner des Benutzers) sendet. Gleiches gilt für die Ergebnisdaten für eine bestimmte Kennung.

Es wird angemerkt, dass mit "Benutzer" im Grunde das Benutzersystem (z.B. ein Terminal) gemeint ist, da für das Netzwerk nur das Benutzersystem relevant ist. In der Praxis kann durchaus mit mehreren Tokens gearbeitet werden. Beispielsweise erfordern einige Netzwerkanwendungen (wie z.B. teamplay) Tokens, die durch eine Authentifizierung aktiviert werden und nach einer gewissen Zeit der Inaktivität ihre Gültigkeit verlieren. Diese Tokens dienen der Kommunikation mit dem entsprechenden Dienst. Es ist bevorzugt, dass der Dienst-Token unabhängig von einem solchen "anwendungsbasierten Token" eine Gültigkeit hat, die der Arbeit mit dem Dienst angemessen ist, z.B. eine Gültigkeit von mindestens einer halben Stunde oder mindesten einer Stunde und/oder maximal von acht Stunden. Das System ist dabei dazu ausgelegt, dass Daten mit dem Dienst-Token unabhängig von anderen Tokens das Netzwerk vom Dienst bis zum Rechner des Benutzers passieren können und besonders bevorzugt auch Daten vom Rechner des Benutzers bis zum Dienst.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine grob schematische Darstellung eines Computertomographiesystems,
Figur 2 eine grob schematische Darstellung der Verarbeitung von Daten durch einen Cloud-Dienst,
Figur 3 eine grob schematische Darstellung der Verarbeitung von Daten durch einen Cloud-Dienst,
Figur 4 ein schematisches Ausführungsbeispiel des erfindungsgemäßen Verfahrens,
Figur 5 ein schematisches Ausführungsbeispiel des erfindungsgemäßen Systems mit einer erfindungsgemäßen Vorrichtung,
Figur 6 ein schematisches Ausführungsbeispiel einer praktischen Umsetzung des erfindungsgemäßen Verfahrens.

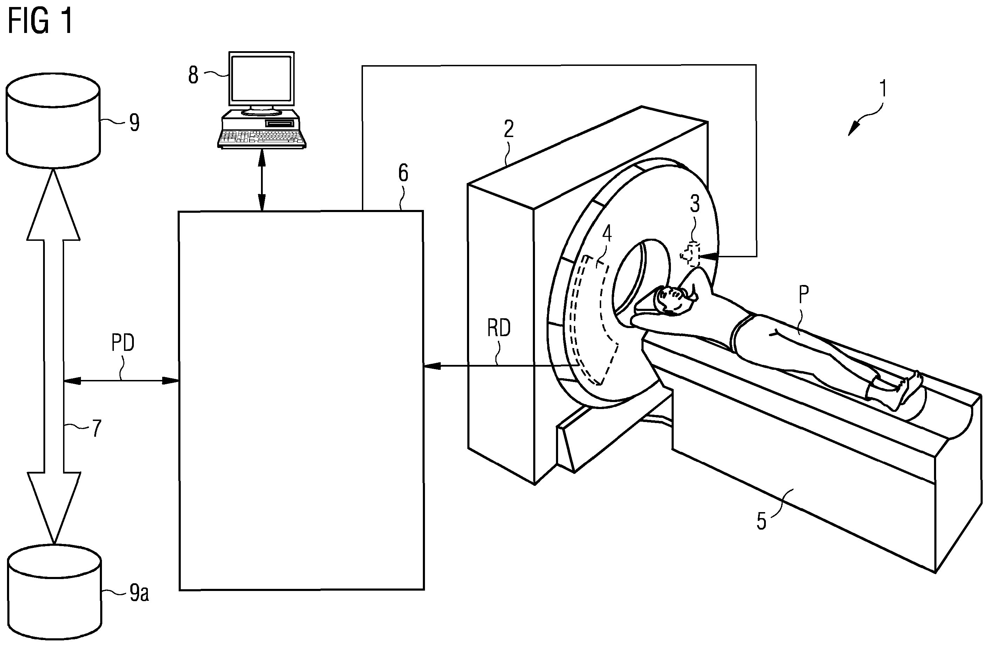

Figur 1 zeigt grob schematisch ein Computertomographiesystem 1 mit einer Steuereinrichtung 6 zur Erzeugung von Bilddaten, die hier als ein Beispiel für Patientendaten angesehen werden. Das Computertomographiesystem 1 weist in üblicher Weise einen Scanner 2 mit einer Gantry auf, in der eine Röntgenquelle 3 rotiert, die jeweils einen Patienten durchstrahlt, welcher mittels einer Liege 5 in einen Messraum der Gantry hineingeschoben wird, so dass die Strahlung auf einen der Röntgenquelle 3 jeweils gegenüberliegenden Detektor 4 trifft.

Die Steuereinrichtung 6 erhält Rohdaten vom Scanner 2, rekonstruiert Bilddaten aus diesen Rohdaten und sendet diese Bilddaten als Patientendaten PD an einen Datenbus 7 weiter. Über einen Terminal 8 kann ein Bediener die Steuereinrichtung 6 und somit das Computertomographiesystem 1 bedienen.

Der Datenbus 7 ermöglicht eine Verbindung zu einem RIS 9a (Radiologieinformationssystem) bzw. PACS 9 (Picture Archiving and Communication System). Der Datenbus kann aber auch eine Verbindung zu einer Netzwerkkomponente in einem medizintechnischen Netzwerk herstellen, mittels derer die Patientendaten PD (zusammen mit einer Kennung K) an einen Cloud-Dienst AI gesendet werden können (s. dazu auch die folgenden Figuren), der in diesen Beispielen ein bevorzugtes Ausführungsbeispiel für einen Server AI bzw. Dienst darstellt und durchaus auch allgemein als Server AI angesehen werden kann.

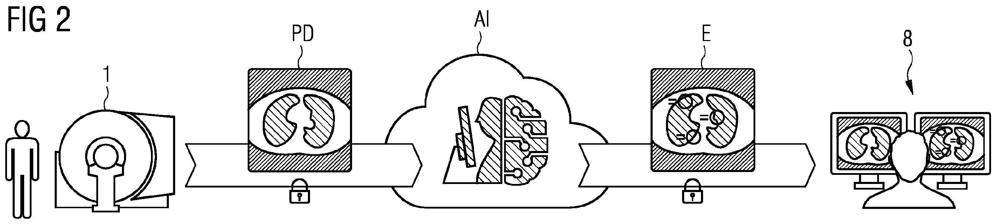

Figur 2 zeigt eine grob schematische Darstellung der Verarbeitung von Patientendaten PD durch einen Cloud-Dienst AI. In diesem Beispiel werden die Patientendaten PD von einem bildgebenden medizintechnischen System 1, hier **z.B.** einem Computertomographiesystem 1, an den Cloud-Dienst AI gesendet, dort bearbeitet und die Ergebnisdaten E (die bearbeiteten Patientendaten) an einen Benutzer bzw. dessen Computersystem gesendet. Dieses Computersystem kann beispielsweise der in Figur 1 dargestellte Terminal 8 mit seiner Anzeigeeinheit 8 sein, der zuzüglich zur Steuerung des CT-Systems 1 auch als Befundungssystem dienen könnte. Die Schlösser unter den Pfeilen, die den Datenfluss verbildlichen sollen, stehen für eine Verschlüsselung der Datenkommunikation, die besonders bevorzugt ist.

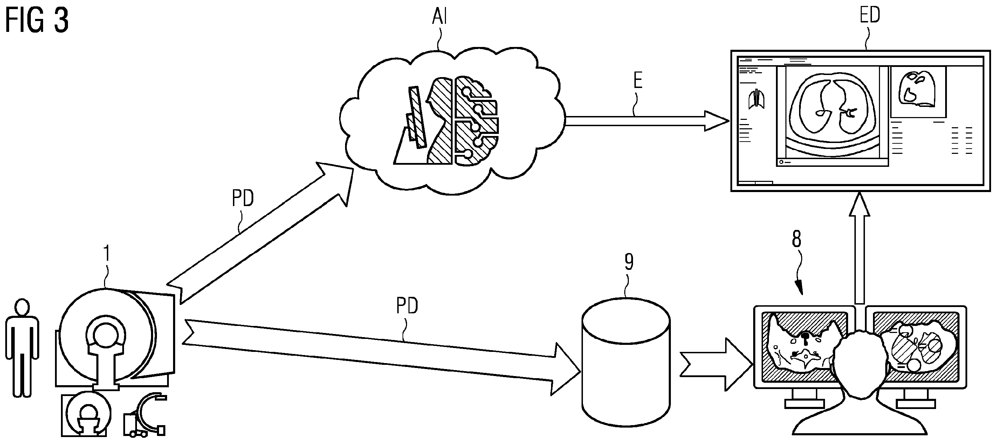

Figur 3 zeigt grob schematisch die Verarbeitung von Patientendaten PD durch einen Cloud-Dienst AI, welcher die Problematik darstellen soll, die der Erfindung zugrunde lag. Auch in diesem Beispiel werden die Patientendaten PD von einem bildgebenden medizintechnischen System 1 an den Cloud-Dienst AI gesendet, aber gleichzeitig auch an ein PACS 9. Der Cloud-Dienst AI (Datenfluss durch Pfeile angedeutet). Der Cloud-Dienst AI darf seine Ergebnisdaten hier jedoch nicht automatisch im PACS 9 abspeichern, so dass diese getrennt vom PACS durch einen Benutzer auf einem Terminal 8 begutachtet werden müssen. Dieser Benutzer greift auf einen (vom PACS aus gesehen) externen Dienst ED zu und begutachtet die Daten dort. Vom Externen Dienst ED aus ist eine Benachrichtigung über den Status der Bearbeitung der Patentendaten problematisch. Außerdem können Probleme im Bereich der Sicherheit auftreten.

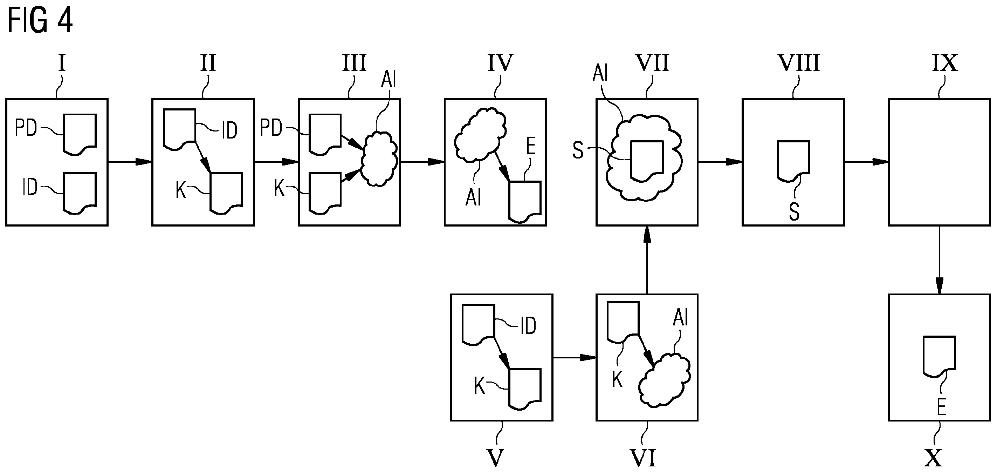

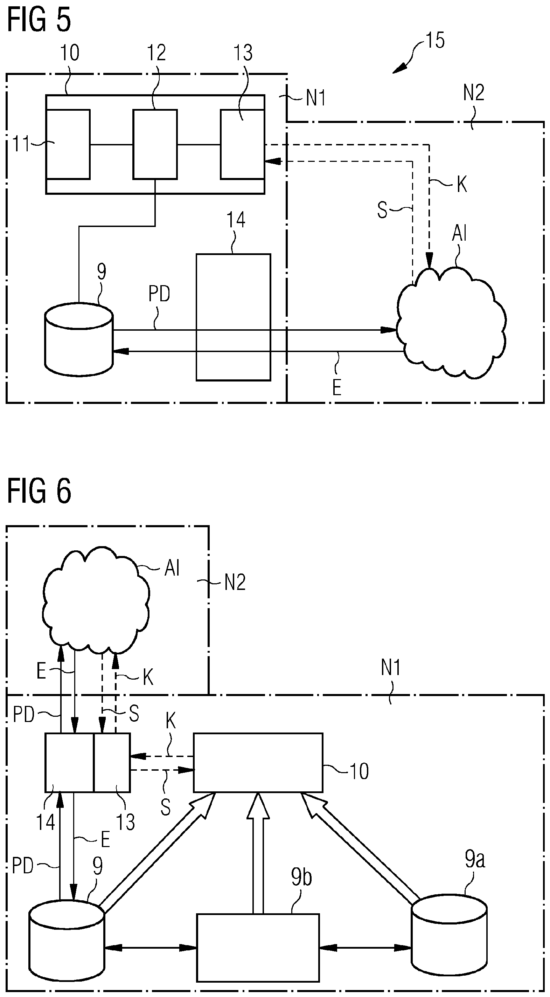

Figur 4 zeigt ein schematisches Ausführungsbeispiel des erfindungsgemäßen Verfahrens zur datentechnischen Kommunikation in einem Netzwerk mit einem ersten (medizintechnischen) Netzwerkbereich N1 und einem zweiten (NoPHI-) Netzwerkbereich N2, an den bestimmungsgemäß Patientendaten PD anonymisiert gesendet werden (siehe dazu auch die Figuren 5 und 6).

In Schritt I erfolgt eine Bereitstellung von medizintechnischen Patientendaten und Identifizierungsdaten ID zur Identifizierung des jeweiligen Patienten P.

In Schritt II wird aus den Identifizierungsdaten ID eine mit diesen Daten sehr eng verknüpfte Kennung K erzeugt, mittels derer eine reversible Anonymisierung der Identifizierungsdaten ID möglich ist.

In Schritt III erfolgt ein Senden von medizintechnischen Patientendaten PD und der jeweils dazugehörigen Kennung K aus dem ersten (medizintechnischen) Netzwerkbereich N1 an einen (Cloud-)Dienst AI im zweiten (NoPHI-) Netzwerkbereich N2.

In Schritt IV werden die vom Cloud-Dienst AI empfangenen Patientendaten PD durch diesen bearbeitet und Ergebnisdaten E erzeugt. Diese Ergebnisdaten E sind selbstverständlich noch mit der jeweiligen Kennung K verknüpft, so dass später eine Zuordnung zum entsprechenden Patienten P möglich ist.

In Schritt V erfolgt eine Bereitstellung von Identifizierungsdaten ID oder Eingabe von Identifizierungsdaten ID zur Identifizierung eines Patienten P durch einen Benutzer. Dies kann vor während oder nach der Bearbeitung von Patientendaten PD durch den Cloud-Dienst AI erfolgen.

In Schritt VI wird aus diesen Identifizierungsdaten ID eine Kennung K ermittelt, welche bei denselben Identifizierungsdaten ID stets identisch ist. Diese Kennung K wird darauf hin automatisch an den Cloud-Dienst AI gesendet.

In Schritt VII erfolgt eine Ermittlung des Status der Bearbeitung von mit dieser Kennung K verbundenen Patientendaten PD, und eine Erstellung einer entsprechenden Statusmitteilung S durch den Cloud-Dienst AI.

In Schritt VIII wird die Statusmitteilung S an den Benutzer gesendet.

In Schritt IX erfolgt eine Interaktion durch den Benutzer zum Empfang der Ergebnisdaten, z.B. ein bestätigender Mausklick auf eine entsprechende Schaltfläche oder einen Link auf dem Bildschirm.

In Schritt X werden die Ergebnisdaten E dann vom Cloud-Dienst AI an den Benutzer zur Begutachtung gesendet. Dieser kann nach der Begutachtung die Ergebnisdaten E in einem Gerät im medizintechnischen Netzwerk N1 abspeichern, z.B. in einem PACS 9. Es können beispielsweise automatisch mit Markierungen versehene Bilddaten (vom Cloud-Dienst gesendet) und von einem Begutachter in einem Browserfenster angeschaut werden.

Figur 5 zeigt ein schematisches Ausführungsbeispiel des erfindungsgemäßen Systems 15 zur datentechnischen Kommunikation mit einer erfindungsgemäßen Vorrichtung 10. Dargestellt ist ein Netzwerk mit einem ersten (medizintechnischen) Netzwerkbereich N1 und einem zweiten (NoPHI-) Netzwerkbereich N2. An den zweiten (NoPHI-) Netzwerkbereich dürfen Patientendaten PD bestimmungsgemäß nur anonymisiert (mit einer Kennung K) gesendet werden. In diesem Netzwerk soll eine erfindungsgemäße Kommunikation stattfinden, wie sie z.B. in Figur 4 genauer beschrieben worden ist. Datenströme werden mit Pfeilen dargestellt.

Das System 15 umfasst eine Einheit ausgelegt zum Bereitstellen von medizintechnischen Patientendaten PD und von Identifizierungsdaten ID zur Identifizierung eines Patienten P. Diese Einheit ist hier in Form eines PACS 9 dargestellt.

Das System 15 umfasst auch eine Netzwerkschnittstelle 14, die zur Kommunikation mit dem Cloud-Dienst dient. Dies bedeutet, dass die Netzwerkschnittstelle 14 als Datenschnittstelle zum Senden von medizintechnischen Patientendaten PD und der dazugehörigen Kennung K aus dem ersten (medizintechnischen) Netzwerkbereich N1 an den Cloud-Dienst AI fungiert.

Oben im medizintechnischen Netzwerk N1 ist eine Vorrichtung 10 dargestellt, die eine Eingabeschnittstelle 11, eine Identifizierungseinheit 12 und eine Datenschnittstelle 13 aufweist.

Die Eingabeschnittstelle 11 ist zur Eingabe von Identifizierungsdaten ID zur Identifizierung eines Patienten durch einen Benutzer ausgelegt. Theoretisch könnte als Eingabeschnittstelle 11 auch das Terminal 8 aus Figur 1 verwendet werden.

Die Identifizierungseinheit 12 ist zur Ermittlung einer mit diesen Identifizierungsdaten ID verknüpften Kennung K ausgelegt. Da eine Kennung eineindeutig mit den Identifizierungsdaten ID verknüpft sein muss, kann die Identifizierungseinheit 12 gleichzeitig als Anonymisierungseinheit 12 verwendet werden. In dem hier dargestellten Beispiel dient die Identifizierungseinheit 12 gleichzeitig als Anonymisierungseinheit 12, bzw. als eine Einheit ausgelegt zum Bereitstellen einer Kennung K, welche mit den Identifizierungsdaten ID von zu sendenden Patientendaten PD verknüpft ist.

Die Datenschnittstelle 13 ist zum Senden der Kennung K an den Cloud-Dienst ausgelegt. Gleichzeitig ist die Datenschnittstelle 13 auch zum Empfang einer Statusmitteilung S des Cloud-Dienstes AI und zur Weitergabe der Statusmitteilung S an den Benutzer ausgelegt. Als Datenschnittstelle 13 könnte theoretisch auch die Netzwerkschnittstelle 14 verwendet werden.

Die Vorrichtung 10 kommuniziert in dem dargestellten Beispiel über die Netzwerkschnittstelle 14 aus dem ersten (medizintechnischen) Netzwerkbereich N1 mit dem Cloud-Dienst AI.

Figur 6 zeigt ein schematisches Ausführungsbeispiel einer praktischen Umsetzung des erfindungsgemäßen Verfahrens. Die Architektur ist ähnlich wie der in Figur 5 dargestellten, mit dem Unterschied, dass die Netzwerkschnittstelle 14 (z.B. ein "teamplay Receiver" einen besonderen Netzwerkdienst (zum Beispiel ein besondere Plugin für teamplay) als Datenschnittstelle 13 aufweist, mit dem die Vorrichtung 10 mit dem Cloud-Dienst AI kommuniziert.

Ist in Figur 5 nur ein Gerät (ein PACS 9) im medizintechnischen Netzwerk N1 eingezeichnet, sind hier drei Geräte vorhanden, ein PACS 9, von dem aus auch die Patientendaten PD an den Cloud-Dienst AI gesendet werden, ein RIS 9a und ein Datenverarbeitungsgerät 9b. Alternativ kann ein Datensatz z.B. auch direkt von einem CT-Scanner (s. z.B. Figur 1) geschickt werden. Wie mit den von den Geräten zur Vorrichtung 10 zeigenden umrandeten Pfeilen angedeutet ist, kann die Vorrichtung 10 hier Informationen der Geräte empfangen. Beispielsweise kann die Vorrichtung 10 vom PACS 9 Hinweise auf die zum Cloud-Dienst AI gesendeten Daten erhalten oder Informationen zu einem Wechsel eines Patienten. Vom RIS 9b kann die Vorrichtung 10 Informationen darüber erhalten, welche Patienten P als nächstes für eine anstehende Begutachtung der automatisch ausgewerteten Daten aufgerufen werden sollen. Vom Datenverarbeitungsgerät 9b kann die Vorrichtung 10 nähere Informationen über die entsprechenden Identifizierungsdaten ID erhalten.

Es wird abschließend noch einmal darauf hingewiesen, dass es sich bei den vorhergehend detailliert beschriebenen Verfahren sowie bei dem dargestellten System lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur datentechnischen Kommunikation in einem Netzwerk (N1, N2) mit einem ersten Netzwerkbereich (N1) und einem zweiten Netzwerkbereich (N2), an den bestimmungsgemäß Patientendaten (PD) anonymisiert gesendet werden, umfassend die Schritte:
- Bereitstellen von medizintechnischen Patientendaten (PD),
- Bereitstellen von Identifizierungsdaten (ID) zur Identifizierung eines Patienten (P),
- Bereitstellen einer Kennung (K), welche mit den Identifizierungsdaten (ID) verknüpft ist,
- Senden von medizintechnischen Patientendaten (PD) und der dazugehörigen Kennung (K) aus dem ersten Netzwerkbereich (N1) an einen Server (AI) im zweiten Netzwerkbereich (N2),
- Bearbeiten der Patientendaten (PD) durch den Server (AI),
- Bereitstellung von Identifizierungsdaten (ID) oder Eingabe von Identifizierungsdaten (ID) zur Identifizierung eines Patienten (P) durch einen Benutzer,
- Ermitteln einer mit diesen Identifizierungsdaten (ID) verknüpften Kennung (K),
- automatisches Senden der Kennung (K) an den Server (AI),
- Ermitteln des Status der Bearbeitung von mit dieser Kennung (K) verbundenen Patientendaten (PD), und Erstellung einer entsprechenden Statusmitteilung (S) durch den Server (AI),
- Senden der Statusmitteilung (S) vom Server (AI) an den Benutzer,
wobei mit oder nach dem Senden der Statusmitteilung (S) an den Benutzer entsprechende Ergebnisdaten (E) vom Server (AI) an den Benutzer gesendet werden,
wobei die vom Benutzer empfangenen Ergebnisdaten (E) an ein Gerät im medizintechnischen Netzwerk (N1) gesendet werden,
wobei zusammen mit der Kennung (K) ein Token von dem Benutzer aus an den Server (AI) gesendet wird und ein Token zusammen mit der Statusmitteilung (S) zurückgesendet wird, wobei das medizintechnische Netzwerk (N1) ein Element umfasst, das diesen Token dem Benutzer zuordnen kann und eine Statusmitteilung (S) und die Ergebnisdaten (E) in Abhängigkeit von den ihnen beigefügten Tokens zu einer bestimmten Station im medizintechnischen Netzwerk (N1) sendet.

2. Verfahren nach dem vorangehenden Anspruch wobei die medizintechnischen Patientendaten (PD) und die Kennung (K) aus dem medizintechnischen Netzwerk (N1) an den Server (AI) gesendet werden, bevorzugt von einem medizintechnischen System (1), einem PACS (9), einem RIS (9a), einem KIS, einer EMR und/oder einem Therapieplanungssystem.

3. Verfahren nach einem der vorangehenden Ansprüche, wobei der Benutzer über ein Gerät und/oder eine Software-Anwendung auf das medizintechnische Netzwerk (N1) zugreift und die Kommunikation zwischen Server (AI) und Benutzer über das medizintechnische Netzwerk (N1) erfolgt.

4. Verfahren nach einem der vorangehenden Ansprüche, wobei die Kommunikation zwischen dem ersten Netzwerkbereich (N1) und dem Server (AI) verschlüsselt ist und bevorzugt mittels einer Software zum Fernzugriff auf Software-Anwendungen, erfolgt,
bevorzugt wobei eine, insbesondere verschlüsselte, Kommunikation zwischen dem Benutzer und dem Server (AI) besonders bevorzugt über eine Software zum Fernzugriff auf Software-Anwendungen erfolgt.

5. Verfahren nach einem der vorangehenden Ansprüche, wobei die Ermittlung einer Kennung (K) anhand von Identifizierungsdaten (ID) mittels einer Lookup-Tabelle, einem Algorithmus oder einer eineindeutigen Funktion durchgeführt wird.

6. Verfahren nach einem der vorangehenden Ansprüche, wobei die Statusmitteilung (S) eine Information über den Zustand der Bearbeitung durch den Server (AI) umfasst,
und im Falle einer fertigen Bearbeitung der Patientendaten (PD) bevorzugt zusätzlich Informationen über ein Ergebnis umfasst,
besonders bevorzugt wobei die Statusmitteilung (S) an den Benutzer ausgegeben wird, insbesondere in Form einer standardisierten Wiedergabe.

7. Vorrichtung (10) zur datentechnischen Kommunikation in einem Netzwerk (N1, N2) mit einem ersten Netzwerkbereich (N1) und einem zweiten Netzwerkbereich (N2), an den bestimmungsgemäß Patientendaten (PD) anonymisiert gesendet werden, wobei die Kommunikation auf medizintechnischen Patientendaten (PD), Identifizierungsdaten (ID) zur Identifizierung eines Patienten (P) und einer mit den Identifizierungsdaten (ID) verknüpften Kennung (K) basiert, umfassend:
- eine Eingabeschnittstelle (11) ausgelegt zur Eingabe von Identifizierungsdaten (ID) zur Identifizierung eines Patienten (P) durch einen Benutzer,
- eine Identifizierungseinheit (12) ausgelegt zum Ermitteln einer mit diesen Identifizierungsdaten (ID) verknüpften Kennung (K),
- eine Datenschnittstelle (13) ausgelegt zum Senden der Kennung (K) an einen Server (AI) in dem zweiten Netzwerkbereich (N2),
- eine Datenschnittstelle (13) ausgelegt zum Empfang einer Statusmitteilung (S) des Servers (AI) und zur Weitergabe der Statusmitteilung (S) an den Benutzer,
wobei die Vorrichtung dazu ausgelegt ist, mit einem Gerät oder Dienst im medizintechnischen Netzwerk (N1) zu kommunizieren,
wobei mit oder nach dem Senden der Statusmitteilung (S) an den Benutzer entsprechende Ergebnisdaten (E) vom Server (AI) an den Benutzer gesendet werden,
wobei die vom Benutzer empfangenen Ergebnisdaten (E) an ein Gerät im medizintechnischen Netzwerk (N1) gesendet werden, wobei zusammen mit der Kennung (K) ein Token von dem Benutzer aus an den Server (AI) gesendet wird und ein Token zusammen mit der Statusmitteilung (S) zurückgesendet wird, wobei das medizintechnische Netzwerk (N1) ein Element umfasst, das diesen Token dem Benutzer zuordnen kann und eine Statusmitteilung (S) und die Ergebnisdaten (E) in Abhängigkeit von den ihnen beigefügten Tokens zu einer bestimmten Station im medizintechnischen Netzwerk (N1) sendet.

8. Vorrichtung nach Anspruch 7, wobei das Gerät oder der Dienst im medizintechnischen Netzwerk (N1) ein Dienst oder Gerät der Gruppe PACS (9), RIS (9a), KIS, EMR, medizintechnisches Messsystem, medizintechnisches Bildgebungssystem und Therapieplanungssystem ist und/oder
wobei die Vorrichtung eine Anonymisierungseinheit (12) zur automatischen Erstellung der Kennung (K) aus Identifizierungsdaten (ID) eines Patienten (P) umfasst, insbesondere nach einer vorgegebenen Tabelle, einem vorgegebenen Algorithmus oder einer vorgegebenen Funktion.

9. Computerprogrammprodukt mit einem Computerprogramm, welches direkt in eine Speicher-einheit eines medizintechnischen Servers oder einer Netzwerkschnittstelle (14) ladbar ist, mit Programmabschnitten, um alle Schritte eines Verfahrens nach einem der Ansprüche 1 bis 6 auszuführen, wenn das Computerprogramm in dem Server oder der Netzwerkschnittstelle (14) ausgeführt wird.

10. Computerlesbares Medium, auf welchem von einer Rechnereinheit einlesbare und ausführbare Programmabschnitte gespeichert sind, um alle Schritte eines Verfahrens nach einem der Ansprüche 1 bis 6 auszuführen, wenn die Programmabschnitte von einer Recheneinheit ausgeführt werden.

## Claims

1. Method for data communication in a network (N1, N2) with a first network area (N1) and a second network area (N2), to which, according to specification, patient data (PD) is sent anonymised, comprising the steps:
- provision of medical patient data (PD),
- provision of identification data (ID) for identification of a patient (P),
- provision of a code (K), which is linked to the identification data (ID),
- sending of medical patient data (PD) and the associated code (K) from the first network area (N1) to a server (AI) in the second network area (N2),
- processing of the patient data (PD) by the server (AI),
- provision of identification data (ID) or input of identification data (ID) for identification of a patient (P) by a user,
- establishing of a code (K) linked to this identification data (ID),
- automatic sending of the code (K) to the server (AI),
- establishing of the status of the processing of patient data (PD) linked to this code (K), and creation of a corresponding status notification (S) by the server (AI),
- sending of the status notification (S) from the server (S) to the user,
wherein corresponding result data (E) is sent from the server (AI) to the user with or after the sending of the status notification (S) to the user,
wherein the result data (E) received by the user is sent to a device in the medical network (N1),
wherein a token is sent from the user to the server (AI) together with the code (K) and a token is sent back together with the status notification (S), wherein the medical network (N1) comprises an element that can assign this token to the user and sends a status notification (S) and the result data (E) as a function of the token enclosed with it to a specific station in the medical network (N1).

2. Method as claimed in the preceding claim, wherein the medical patient data (PD) and the code (K) are sent from the medical network (N1) to the server (AI), preferably from a medical system (1), a PACS (9), an RIS (9a), a HIS, an EMR and/or a therapy planning system.

3. Method as claimed in one of the preceding claims, wherein the user accesses the medical network (N1) via a device and/or a software application and communication between server (AI) and user is undertaken via the medical network (N1).

4. Method as claimed in one of the preceding claims, wherein communication between the first network area (N1) and the server (AI) is encrypted and is preferably undertaken by means of software for remote access to software applications,
wherein there is preferably, in particular encrypted, communication between the user and the server (AI), especially preferably via software for remote access to software applications.

5. Method as claimed in one of the preceding claims, wherein the establishing of a code (K) is carried out on the basis of identification data (ID) by means of a lookup table, an algorithm or a unique function.

6. Method as claimed in one of the preceding claims, wherein the status notification (S) comprises information about the status of the processing by the server (AI),
and in the event of processing of the patient data (PD) having been finished, preferably additionally comprises information about a result,
especially preferably wherein the status notification (S) is output to the user, in particular in the form of a standardised representation.

7. Apparatus (10) for data communication in a network (N1, N2) with a first network area (N1) and a second network area (N2), to which, according to specification, patient data (PD) is sent anonymised, wherein the communication is based on medical patient data (PD), identification data (ID) for identification of a patient (P) and a code (K) linked to the identification data (ID), comprising:
- an input interface (11) designed for entry of identification data (ID) for identification of a patient (P) by a user,
- an identification unit (12) designed for establishing a code (K) linked to this identification data (ID),
- a data interface (13) designed for sending the code (K) to a server (AI) in the second network area (N2),
- a data interface (13) designed for receiving a status notification (S) of the server (AI) and for forwarding the status notification (S) to the user,
wherein the apparatus is designed to communicate with a device or service in the medical network (N1),
wherein corresponding result data (E) is sent from the server (AI) to the user with or after the sending of the status notification (S) to the user,
wherein the result data (E) received by the user is sent to a device in the medical network (N1),
wherein a token is sent from the user to the server (AI) together with the code (K) and a token is sent back together with the status notification (S), wherein the medical network (N1) comprises an element, that can assign this token to the user and sends a status notification (S) and the result data (E) as a function of the token enclosed with it to a specific station in the medical network (N1).

8. Apparatus as claimed in claim 7, wherein the device or the service in the medical network (N1) is a service or device of the group PACS (9), RIS (9a), HIS, EMR, medical measuring system, medical imaging system and therapy planning system and/or
wherein the apparatus comprises an anonymisation unit (12) for automatic creation of the code (K) from identification data (ID) of a patient (P), in particular according to a predetermined table, a predetermined algorithm or a predetermined function.

9. Computer program product with a computer program, which is able to be loaded directly into a memory unit of a medical server or a network interface (14), with program sections for carrying out all steps of a method as claimed in one of claims 1 to 6 when the computer program is executed in the server or the network interface (14).

10. Computer-readable medium, on which program sections able to be read into and executed by a computer unit are stored, in order to carry out all steps of a method as claimed in one of claims 1 to 6, when the program sections are executed by a computer unit.

## Revendications

1. Procédé de communication technique de données dans un réseau (N1, N2) ayant une première partie (N1) de réseau et une deuxième partie (N2) de réseau, auxquelles on envoie de manière anonyme des données (PD) de patient conformément aux prescriptions, comprenant les stades :
- se procurer des données (PD) de technique médicale du patient,
- se procurer des données (ID) d'identification pour l'identification du patient (P),
- se procurer une caractéristique (K), qui est combinée aux données (ID) d'identification,
- envoyer des données (PD) de technique médicale du patient et de la caractéristique (K), qui y est associée, de la première partie (N1) du réseau à un serveur (AI) dans la deuxième partie (N2) du réseau,
- traiter des données (PD) du patient par le serveur (AI),
- mettre à disposition des données (ID) d'identification ou entrer des données (ID) d'identification pour l'identification d'un patient (P) par un utilisateur,
- établir une caractéristique (K) combinée à ces données (ID) d'identification,
- envoyer automatiquement la caractéristique (K) au serveur (AI),
- établir le statut du traitement de données (PD) du patient reliées à cette caractéristique (K) et créer une notification (S) correspondante de statut par le serveur (AI),
- envoyer la notification (S) de statut du serveur (AI) à l'utilisateur,
dans lequel, avec ou après l'envoi de la notification (S) de statut à l'utilisateur, on envoie des données (E) de résultat correspondantes du serveur (AI) à l'utilisateur,
dans lequel on envoie les données (E) de résultat reçues par l'utilisateur à un appareil dans le réseau (N1) de technique médicale,
dans lequel ensemble avec la caractéristique (K), on envoie un jeton de l'utilisateur au serveur (AI) et on renvoie un jeton ensemble avec la notification (S) de statut, dans lequel le réseau (N1) de technique médicale comprend un élément, qui peut affecter ce jeton à l'utilisateur et envoie une notification (S) de statut et les données (E) de résultat en fonction des jetons, qui leur sont attribuées, à un poste déterminé dans le réseau (N1) de technique médicale.

2. Procédé suivant l'une des revendications précédentes, dans lequel on envoie les données (PD) de technique médicale du patient et la caractéristique (K) du réseau (N1) de technique médicale au serveur (AI), de préférence d'un système (1) de technique médicale, d'un PACS (9), d'un RIS (9a), d'un KIS, d'un EMR et/ou d'un système de planification de thérapie.

3. Procédé suivant l'une des revendications précédentes, dans lequel l'utilisateur accède par un appareil et/ou par une application logicielle au réseau (N1) de technique médicale et la communication entre serveur (AI) et utilisateur s'effectue par le réseau (N1) de technique médicale.

4. Procédé suivant l'une des revendications précédentes, dans lequel la communication entre la première partie (N1) du réseau et le serveur (AI) est chiffrée et s'effectue de préférence au .
moyen d'un logiciel pour l'accès à distance à des applications logicielles,
dans lequel, de préférence, il s'effectue une communication, en particulier chiffrée entre l'utilisateur et le serveur (AI), de manière particulièrement préférée, par un logiciel pour l'accès à distance à des applications logicielles.

5. Procédé suivant l'une des revendications précédentes, dans lequel on effectue l'établissement d'une caractéristique (K) à l'aide de données (ID) d'identification au moyen d'une table de consultation, d'un algorithme ou d'une fonction univoque.

6. Procédé suivant l'une des revendications précédentes, dans lequel la notification (S) de statut comprend une information sur l'état du traitement par le serveur (AI),
et, dans le cas d'un traitement fini des données (PD) du patient comprend, de préférence supplémentairement, des informations sur un résultat,
dans lequel, d'une manière particulièrement préférée, on donne la notification (S) de statut à l'utilisateur, en particulier sous la forme d'un compte-rendu normalisé.

7. Dispositif (10) de communication technique de données dans un réseau (N1, N2) ayant une première partie (N1) de réseau et une deuxième partie (N2) de réseau, auxquelles on envoie de manière anonyme des données (PD) de patient conformément aux prescriptions, dans lequel la communication repose sur des données (PD) de patient de technique médicale, des données (ID) d'identification pour l'identification d'un patient (P) et d'une caractéristique (K) combinée aux données (ID) d'identification, comprenant :
- une interface (11) d'entrée conçue pour l'entrée de données (ID) d'identification pour l'identification d'un patient (P) par un utilisateur,
- une unité (12) d'identification conçue pour l'établissement d'une caractéristique (K) combinée à ces données (ID) d'identification,
- une interface (13) de données conçue pour l'envoi de la caractéristique (K) à un serveur (AI) dans la deuxième partie (N2) du réseau,
- une interface (13) de données conçue pour la réception d'une notification (S) de statut du serveur (AI) et pour l'acheminement de la notification (S) de statut à l'utilisateur,
dans lequel le dispositif est conçu pour communiquer avec un appareil ou un service dans le réseau (N1) de technique médicale, dans lequel, avec ou après l'envoi de la notification (S) de statut, on envoie des données (E) de résultat correspondantes à l'utilisateur du serveur (AI) à l'utilisateur,
dans lequel on envoie des données (E) de résultat reçues par l'utilisateur à un appareil dans le réseau (N1) de technique médicale, dans lequel ensemble avec la caractéristique (K), on envoie un jeton de l'utilisateur au serveur (AI) et on renvoie un jeton ensemble avec la notification (S) de statut, dans lequel le réseau (N1) de technique médicale comprend un élément, qui peut associer ce jeton à l'utilisateur et envoie une notification (S) de statut et les données (E) de résultat en fonction des jetons, qui y sont joints, à un poste déterminé dans le réseau (N1) de technique médicale.

8. Dispositif suivant la revendication 7,
dans lequel l'appareil ou le service dans le réseau (N1) de technique médicale est un service ou un appareil du groupe PACS (9), RIS (9a), KIS, EMR, système de mesure de technique médicale, système d'imagerie de technique médicale et système de planification de thérapie,
et/ou
dans lequel le dispositif comprend une unité (12) d'anonymisation pour l'établissement automatique de la caractéristique (K) à . partir de données (ID) d'identification d'un patient (P), en particulier suivant une table donnée à l'avance, d'un algorithme donné à l'avance ou d'une fonction donnée à l'avance.

9. Produit de programme d'ordinateur comprenant un programme d'ordinateur, qui peut être chargé directement dans une unité de mémoire d'un serveur de technique médicale ou d'une interface (14) de réseau comprenant des parties de programme pour exécuter tous les stades d'un procédé suivant l'une des revendications 1 à 6, lorsque le programme d'ordinateur est exécuté dans le serveur ou l'interface (14) de réseau.

10. Support déchiffrable par ordinateur, sur lequel sont mises en mémoire des parties de programme pouvant être lues et réalisées par une unité informatique, afin d'exécuter tous les stades d'un procédé suivant l'une des revendications 1 à 6, lorsque les parties de programme sont exécutées par une unité informatique.
